# EUROPEAN PATENT APPLICATION

(11) **EP 1 681 067 A1**
(43) Date of publication of application: **19.07.2006**
(21) Application number: 04792815.5
(22) Date of filing: 15.10.2004
(51) Int. Cl.: A61L 27/00, A61K 48/00

(54) **IMPLANT FOR REGENERATING BONE OR CARTILAGE WITH THE USE OF TRANSCRIPTIONAL FACTOR**

(30) Priority: 15.10.2003 JP 2003035505
(71) Applicant: Japan Science and Technology Agency, Kawaguchi-shi, Saitama 332-0012 (JP)
(72) Inventor: KOJIMA, Hiroko, Tsukuba-shi, Ibaraki 305-0031 (JP); UEMURA, T., Nat. Inst of Adv. Ind. Science & Techn, Tsukuba-shi, Ibaraki 305-8566 (JP)
(74) Representative: Vossius & Partner
(86) International application number: PCT/JP2004/015673
(87) International publication number: WO 2005/035014

(57) **Abstract**

This invention provides an implant that enable satisfactory bone/cartilage regeneration by allowing sustained release of virus vectors carrying the transcription factor gene *in vivo* and persistent maintenance of transcription factor activity.

SEQUENCE LISTING

<110> Japan Science and Technology Agency
National Institute of Advanced Industrial Science and Technology

<120> Implant for regeneration of bone/cartilage tissue by using transcription factor

<130> PH-2275-PCT

<150> JP 2003-355505
<151> 2003-10-15

<160> 3

<170> PatentIn Ver. 2.1

<210> 1
<211> 1791
<212> DNA
<213> Mus musculus

<220>
<221> CDS

<400> 1
atgcttcatt cgcctcacaa acaaccacag aaccacaagt gcggtgcaaa ctttctccag 60
gaagactgca agaaggctct ggcgtttaaa tggttaatct ctgcaggtca ctaccagcca 120
ccgagaccaa ccgagtcatt taaggctgca agcagtattt acaacagagg gcacaagttc 180
tatctggaaa aaaaaggagg gactatggcg tcaaacagcc tcttcagcgc agtgacaccg 240
tgtcagcaaa gcttcttttg ggatccgagc accagccggc gcttcagccc cccctccagc 300

agcctgcagc ccggcaagat gagcgacgtg agcccggtgg tggctgcgca gcagcagcaa 360
cagcagcagc agcagcagca acagcagcag caacaacagc aacagcaaca acagcagcag 420
cagcagcagc agcaggaggc ggccgcagca gcagcggcgg cagcggcggc ggcagcagcg 480
gcggcggccg cagtgccccg attgaggccg ccgcacgaca accgcaccat ggtggagatc 540
atcgcggacc acccggccga actggtccgc accgacagtc ccaacttcct gtgctccgtg 600
ctgccctcgc actggcggtg caacaagacc ctgcccgtgg ccttcaaggt tgtagccctc 660
ggagaggtac cagatgggac tgtggttacc gtcatggccg ggaatgatga gaactactcc 720
gccgagctcc gaaatgcctc cgctgttatg aaaaaccaag tagccaggtt caacgatctg 780
agatttgtgg gccggagcgg acgaggcaag agtttcacct tgaccataac agtcttcaca 840
aatcctcccc aagtggccac ttaccacaga gctattaaag tgacagtgga cggtccccgg 900
gaaccaagaa ggcacagaca gaagcttgat gactctaaac ctagtttgtt ctctgatcgc 960
ctcagtgatt tagggcgcat tcctcatccc agtatgagag taggtgtccc gcctcagaac 1020
ccacggccct ccctgaactc tgcaccaagt ccttttaatc cacaaggaca gagtcagatt 1080
acagatccca ggcaggcaca gtcttcccca ccgtggtcct atgaccagtc ttacccctcc 1140
tatctgagcc agatgacatc cccatccatc cactccacca cgccgctgtc ttccacacgg 1200
ggcaccgggc tacctgccat cactgacgtg cccaggcgta tttcagatga tgacactgcc 1260
acctctgact tctgcctctg gccttcctct ctcagtaaga agagccaggc aggtgcttca 1320
gaactgggcc ctttttcaga ccccaggcag ttcccaagca tttcatccct cactgagagc 1380
cgcttctcca acccacgaat gcactaccca gccaccttta cctacacccc gccagtcacg 1440
tcaggcatgt ccctcggcat gtccgccacc actcactacc acacgtacct gccaccaccc 1500
taccccggct cttcccaaag ccagagtgga cccttccaga ccagcagcac tccatatctc 1560
tactatggta cttcgtcagc atcctatcag ttcccaatgg tacccggggg agaccggtct 1620
ccttccagga tggtcccacc atgcaccacc acctcgaatg gcagcacgct attaaatcca 1680
aatttgccta accagaatga tggtgttgac gctgacggaa gccacagcag ttccccaact 1740
gttttgaatt ctagcggcag aatggatgag tctgtttggc ggccatattg a 1791

<210> 2
<211> 22
<212> DNA

<213> Artificial Sequence

<220>
<223> Description of Artificial Sequence:primer

<400> 2
atgcttcatt cgcctcacaa ac                                                22

<210> 3
<211> 21
<212> DNA
<213> Artificial Sequence

<220>
<223> Description of Artificial Sequence:primer

<400> 3
tctgtttggc ggccatattg a                                                 21

## Description

### Technical Field

The present invention relates to an implant utilizing transcription factors. More particularly, the present invention relates to an implant that enables satisfactory bone/cartilage regeneration by allowing continuous release of virus vectors carrying the transcription factor genes *in vivo* for maintaining the transcription factor activity.

### Background Art

Bones are tissues with a limited regeneration capacity, and their repair thereof requires transplantation of autologous tissues or filling or replacement with artificial implants. Use of autologous bones, however, imposes a heavy burden on a patient, and the amounts thereof that can be obtained are limited. Also, strength, mechanical properties, and bioadaptability as good as those of biological bones cannot be expected from artificial implants.

Meanwhile, research regarding "regenerative medicine" has made progress. In regenerative medicine, cells harvested from a body are cultured *in vitro* and organized to reconstruct tissues that are as similar as possible to those in vivo. The formed tissues are then returned to the body. If such research bears results, it could give rise to the most ideal therapy for repairing tissue defects.

The field of regenerative medicine suffers from several critical problems. For example, cells need to proliferate and differentiate to result in tissues of interest as rapid as possible by *in vitro* cell culture. Also, the transplanted tissues need to be quickly adjusted to the defects, and be reconstructed after *in vivo* transplantation. In order to deal with such issues, several techniques in which cytokines (humoral factors) responsible for cell differentiation are directly added to cells are known. Examples of known techniques include: a technique of culturing bone marrow cells in a collagen sponge adsorbed with TGF-β1 (JP Patent Publication (Kokai) No. 2001-316285 A) and a material for regenerating cartilage tissues using a collagen-cartilage cell composite comprising bFGF (JP Patent Publication (Kokai) No. 8-3199 A (1996)). In these techniques in which the growth factors are directly added to cells, however, the growth factors rapidly diffuse throughout the body, and their activity cannot be maintained for a sufficiently long time. A method wherein growth factor genes are directly introduced into cells by lipofection method has been attempted. Such technique has been somewhat effective in established cell lines, although the transfection efficiency was found to be almost 0 in primary culture cells.

Recently, many researchers have found that transcription factors, particularly transcription factors of runt and Helix-Loop-Helix (HLH) types, play important roles in osteoblast differentiation. For example, a runt transcription factor, Pebp2alphaA (Pebp2αA)/Cbfa1, and HLH transcription factors, such as Scleraxis, Id-1, and I-mfa, have been reported to play important roles in osteoblast differentiation (Komori, T. et al., 1997, Cell 89, pp. 755-764; Acampora, D. et al., 1999, Development 126, pp. 3795-3809; Tribioli, C. et al., 1999, Development 126, pp. 5699-5711; Satokata, I. et al., 2000, Nature Genet. 24, pp. 391-395; Cserjesi, P. et al., 1995, Development 121, pp. 1099-1110; Ng, L.J. et al., 1997, Dev. Biol. 183, pp. 108-121).

The present inventors have reported that regeneration of bone/cartilage tissue of good quality can be realized by culturing and organizing osteoblasts into which transcription factor (Cbfa1) genes have been introduced with the use of biodegradable scaffold materials such as β-TCP (H. Kojima, T. Uemura, 2002, Molecular Biology of the Cell, Vol. 13 supplement, p. 543a; Toshimasa Uemura, Hiroko Kojima, 2002, Tissue Engineering, Vol. 8, No. 6, p. 1129) and transplanting the composite into the body (WO 03/011343). This technique is excellent in terms of feasibility of effective bone/cartilage regeneration. However, a procedure comprising isolation of cells from a patient, tissue construction *ex vivo,* and transplantation of the cells to the body is difficult in clinics.

### Disclosure of the Invention

An object of the present invention is to provide a simpler and safer means for bone/cartilage regeneration in the fields of orthopedics and dentistry.

The present inventors have conducted concentrated studies in order to attain the above object. As a result, they have discovered a method that enables continuous release of virus vectors for introducing transcription factor genes *in vivo* with the use of a bioadaptable material such as β-TCP or hydroxyapatite. The present inventors demonstrated that such method enables bone/cartilage regeneration with the use of a small amount of virus vectors to an extent equal to the regeneration implemented by the method involving the transplantation of cells into which transcription factor genes have been introduced. This has led to the completion of the present invention.

Specifically, the present invention relates to an implant for bone/cartilage regeneration consisting of bioadaptable materials comprising the adenoviral or retroviral vectors into which the genes of osteo-/chondro-inducible transcription factors have been introduced.

The implant of the present invention can comprise, as osteo-/chondro-inducible transcription factors, at least one member selected from the group consisting of Cbfa1, Dlx-5, Bapx1, Msx2, Scleraxis, and Sox9, for example. Cbfa1 is particularly preferable.

Examples of bioadaptable materials that can be used for the implant of the present invention include hydroxyapatite, α-TCP, β-TCP, collagen, polylactic acid, hyaluronic acid, polyglycolic acid, and a hybrid of any thereof. β-TCP is particularly preferable.

According to the present invention, activity of transcription factors that promotes bone/cartilage regeneration *in vivo* is maintained for long time, and damaged bone/cartilage can be satisfactorily regenerated.

Hereafter, the present invention is described in greater detail.

The present invention relates to an implant consisting of bioadaptable materials adsorbing virus vectors carrying the genes of osteo-/chondro-inducible transcription factors. Such implants are used for bone/cartilage regeneration in the fields of dentistry and orthopedics, such as regeneration of alveolar bones or thighbones.

### 1. Transcription factors

The transcription factors employed in the present invention are the osteo-/chondro-inducible transcription factors that induce immature cells to differentiate into bone/cartilage cells. Examples thereof include Cbfa1, Dlx-5, Bapx1, Msx2, Scleraxis, and Sox9. Cbfa1 is a transcription factor that was cloned by Ogawa et al. at Kyoto University in 1993 and proved to be indispensable for the induction of differentiation of mesenchymal stem cells into osteoblasts by Komori et al. at Osaka University (Komori, T. et al., 1997, Cell 89, 755-764). This Cbfa1 has two isoforms, til-1 and pebp2αA. Dlx-5 is a homolog of the Drosophila distalless (Dll) gene, and it is a transcription factor associated with the ossification of perichondrium and endosporium (Acampora, D. et al., 1999, Development 126, 3795-3809). Bapx1 is a homolog of the Drosophila bagpipe homeobox gene, it is particularly associated with differentiation of mesenchymal stem cells into cartilage cells in the spinal cord, and it is considered to be a regulatory gene of the Cbfa1 gene (Tribioli, C. et al., 1999, Development 126, 5699-5711). Msx2 is a homolog of the Drosophila muscle segment homeobox (Msh) gene, it is associated with the ossification of calvaria, and it is considered to be a regulatory gene of the Cbfa1 gene (Satokata, I. et al., 2000, Nature Genet. 24, 391-395). Scleraxis is a transcription factor that is associated with induction of differentiation of mesenchymal stem cells into cartilage cells or connective tissues (Cserjesi, P. et al., 1995, Development 121, 1099-1110). Sox9 is expressed in cartilage and it regulates expression of the genes associated with cartilage differentiation of, for example, type II collagen (Ng, L. J. et al., 1997, Dev. Biol. 183, 108-121).

The origins of the transcription factors that are used in the present invention are not particularly limited, although the use of transcription factors originating from the mammalians that are targets of transplantation is preferable. When regeneration of human bone/cartilage is intended, accordingly, use of transcription factors obtained from a human is preferable. When regeneration of murine bone/cartilage is intended, accordingly, use of transcription factors obtained from a mouse is preferable.

In this description, osteo-/chondro-inducible transcription factors, such as Cbfa1, Dlx-5, Bapx1, Msx2, Scleraxis, and Sox9, include orthologs thereof. The term "orthologs" used herein refers to the orthologous genes, and these genes have evolutionarily the same origin and similar structures and functions, although they are of different types. The nucleotide sequences of such genes are already known and information on the same is available from the public databases of GenBank or the like. For example, the nucleotide sequences of the Cbfa1 genes derived from murine osteoblasts are registered at GenBank under the accession numbers AF010284, AF005936, and the like. The nucleotide sequences of human Cbfa1 are registered at GenBank under the accession numbers AH005498, NM004348, and L40992 at GenBank. Also, human Dlx-5 is registered under the accession number AK023493, human Bapx1 is registered under the accession number NM_001189, human Msx2 is registered under the accession number D31771, human Scleraxis is registered under the accession number BK000280, and human Sox-9 is registered under the accession number Z46629 at GenBank.

The genes of the osteo-/chondro-inducible transcription factors used in the present invention can be prepared by conventional techniques with the use of the aforementioned sequence information. Specifically, total RNA or mRNA is extracted from osteoblasts, and the primers prepared based on the known sequences are used to amplify cDNA of the target transcription factor by PCR.

### 2. Preparation of viral vector

The above-mentioned adenoviral or retroviral vectors can be constructed according to a known technique. For example, an adenoviral vector can be constructed according to the method of Miyake et al. (Miyake, S. et al., Proc. Natl. Acad. Sci. 93: 1320-1324, 1993). Alternatively, a commercially available kit, such as the Adenovirus Cre/loxP Kit (Takara Shuzo Co., Ltd.), can be used. This kit is used for constructing a recombinant adenoviral vector utilizing a regulation system of gene expression (Kanegae Y. et al., 1995, Nucl. Acids Res. 23, 3816) that employs Cre recombinase of P1 phage and its recognition sequence, loxP. This kit can be used to simply construct a recombinant adenoviral vector carrying the target transcription factor gene. The multiplicity of infection (moi) of adenovirus infection is 10 or more, preferably 50 to 200, and more preferably around 100 (approximately 80 to 120).

### 3. Bioadaptable material

The bioadaptable materials used in the present invention are not particularly limited as long as they are adaptable to the body. Examples thereof include hydroxyapatite, α-TCP, β-TCP, collagen, polylactic acid, hyaluronic acid, and polyglycolic acid. These materials may be used solely or in combinations of two or more.

The aforementioned bioadaptable materials are preferably porous (or particle aggregates) with a large effective surface area for adsorption, so that the material can adsorb large quantities of viral vectors. In the present description, the term "porous" refers to a porosity of 30% or higher. The pore size of the bioadaptable materials according to the present invention is not particularly limited, although a diameter of approximately 50 µm to 500 µm is preferable. The initial strength is critical for implants for hard tissues, such as bones. Porous ceramics such as hydroxyapatite and β-TCP have sufficient strength and thus are preferable for the bioadaptable materials according to the present invention. More preferably, the bioadaptable materials are biodegradable and adsorbable *in vivo* after bone regeneration.

β-TCP is a biodegradable porous ceramic with a sufficient strength and thus is particularly preferable as the bioadaptable material according to the present invention. The compressive strength of porous β-TCP is approximately 3 M Pa, which is lower than that of biological bones (approximately 7 M Pa in the case of cancellous bone). Such strength is sufficient for clinical use. Further, β-TCP is gradually degraded *in vivo* and it releases calcium ions and phosphate ions to provide an environment where the synthesis of hydroxyapatite mediated by osteoblasts is facilitated. Specifically, calcium ions and phosphate ions released from β-TCP enable the synthesis of hydroxyapatite mediated by surrounding osteoblasts. The synthesized hydroxyapatite serves as the bone constituent and it is converted to hard bone tissue in due course. If the bioadaptable material comprises adsorbed thereon virus vectors carrying transcription factor genes, and if the environment surrounding such bioadaptable material is suitable for ossification as in the case of the present invention, new bones mainly composed of hydroxyapatite are easily formed at the site of β-TCP degradation. Specifically, β-TCP not only functions as a carrier for virus delivery or as a scaffold for bone formation, but it also promotes bone formation.

### 4. Incorporation of vectors into bioadaptable materials

Vectors may be incorporated into bioadaptable materials via any technique without particular limitation; however, it is preferable that vectors be incorporated as homogenously as possible. Vectors may be chemically bound to the bioadaptable materials, or they may be merely physically adsorbed thereon. In the case of β-TCP, for example, the bioadaptable materials are soaked in a vector-containing solution (i.e., a medium) to allow β-TCP to homogenously adsorb vectors. Such soaking may be carried out under reduced pressure according to need, so that the vector-containing solution is sufficiently diffused throughout the bioadaptable materials. For example, a viral vector solution (1 x 10⁸ to 10⁹ pfu/ml) is diluted with an adequate serum-free solvent (e.g., isotonic sodium chloride solution) or medium, and a block of bioadaptable materials is soaked therein. The inside of the block is degasified under a reduced pressure of 100 to 150 mmHg to allow the viral vector solution to thoroughly fill the block.

The vector-adsorbed bioadaptable materials are preferably transplanted into the body within several hours. When the bioadaptable materials of the present invention are implanted or injected into the body, they gradually release the viral vectors carrying transcription factor genes over the period of the time required for bone regeneration. Thus, satisfactory bone regeneration can be realized.

### 5. Others

The configurations and shapes of the implant of the present invention are not particularly limited. The implant can take any desired configurations or shapes, such as sponges, meshes, unwoven fabric products, discs, films, sticks, particles, or pastes. The implant of the present invention may be used in combination with other materials. For example, β-TCP granules carrying viruses adsorbed thereon can be mixed with hydroxyapatite granules, and the resultant can be used as a bone filler. These configurations and shapes may be suitably selected depending on the applications of the implant.

The implant of the present invention can suitably contain other components in addition to the bone/cartilage tissues and scaffolds within the scope of the present invention. Examples of such components include: growth factors such as basic fibroblast growth factors (bFGF), platelet-derived growth factors (PDGF), insulin and insulin-like growth factors (IGF), hepatocyte growth factors (HGF), glial-derived neurotrophic factors (GDNF), neurotrophic factors (NF), hormones, cytokines, bone morphogenetic factors (BMP), transforming growth factors (TGF), and vascular endothelial growth factors (VEGF); bone morphogenetic proteins; inorganic salts such as St, Mg, Ca, and CO₃; organic substances such as citric acid and phospholipids; and drugs.

The implant of the present invention is highly compatible with bones. Thus, they can be integrated with biological bones and can then repair bone defects immediately after they have been transplanted into a body.

### Brief Description of the Drawings

Fig. 1 schematically shows a method of experimentation concerning subcutaneous transplantation into a rat's back.
Fig. 2 is a photograph showing an image of hematoxylin-eosin staining in the experimentation concerning subcutaneous transplantation into a rat's back (3 weeks after transplantation).
Fig. 3 schematically shows a method of experimentation concerning transplantation into rat femur bone defects.
Fig. 4 is a photograph showing an image of hematoxylin-eosin staining in the experimentation concerning transplantation of the β-TCP block into rat femur bone defects (2 weeks after transplantation).
Fig. 5 is a photograph showing an image of hematoxylin-eosin staining in the experimentation concerning transplantation of the β-TCP block into rat femur bone defects (6 weeks after transplantation).
Fig. 6 is a photograph showing an image of hematoxylin-eosin staining in the experimentation concerning transplantation of the HA block into rat femur bone defects (3 weeks after transplantation).
Fig. 7 is a photograph showing images of hematoxylin-eosin staining of the β-TCP block and of the HA block after transplantation into rat femur bone defects (3 weeks after transplantation).
Fig. 8 is a photograph showing images of hematoxylin-eosin staining of the virus-adsorbed block and of the virus-infected cell-adsorbed block after transplantation of the β-TCP blocks into rat femur bone defects (8 weeks after transplantation).
Fig. 9 is a photograph showing an image of hematoxylin-eosin staining in the experimentation concerning subcutaneous transplantation of the OPLA composites into a rat's back (20 days and 8 weeks after transplantation).
Fig. 10 is a photograph showing an image of osteocalcin immunostaining in the experimentation concerning subcutaneous transplantation of the OPLA composites into a rat's back (20 days after transplantation).
Fig. 11 is a photograph showing images of hematoxylin-eosin staining in the experimentation concerning transplantation of the OPLA composites into rat femur bone defects (10 days and 20 days after transplantation).
Fig. 12 is a photograph showing images of hematoxylin-eosin staining in the experimentation concerning transplantation of OPLA composites into rat femur bone defects (3 weeks after transplantation).

This description includes a part or all of the contents as disclosed in the description of Japanese Patent Application No. 2003-355505, which is a priority document of the present application.

### Preferred Embodiments of the Invention

### Example 1: Study of sustained release of a viral vector utilizing bioadaptable material carrier (1)

### β-TCP block and HA block

### 1. Preparation of adenoviral vector

cDNA was synthesized from the total RNA isolated from murine osteoblasts using AMV reverse transcriptase, and PCR was performed with the use of the resulting cDNA as a template and primers specific to Cbfa1 cDNA (GenBank Accession No. AF010284: SEQ ID NO: 1) to obtain Cbfa1 cDNA.
Sense primer: 5'-ATGCTTCATTCGCCTCACAAAC-3' (SEQ ID NO: 2)
Antisense primer: 5'-TCTGTTTGGCGGCCATATTGA-3' (SEQ ID NO: 3)

A large quantity of Cbfa1 cDNA was prepared via cloning into the TA cloning vector (pCR 11-TOPO, Invitrogen). Cbfa1 cDNA was cleaved with the SpeI and EcoRV restriction enzymes, blunt-ended, and then inserted into the Swal site of the pAxCALNLw cosmid vector with the use of the Adenovirus Cre/loxP kit (6151, Takara Shuzo Co., Ltd.) to prepare a recombinant adenoviral vector in accordance with the instructions of the kit. The titer of the resulting virus was approximately 10¹¹ PFU/ml, and it was confirmed that the infection efficiency was very high.

### 2. Sampling and culture of osteoblasts

Rat bone marrow osteoblasts (RBMO) were obtained from a thighbone of a 6-week-old Fisher rat (male) in accordance with the method of Maniatopoulos et al. (Maniatopoulos, C., Sodek, J., and Melcher, A. H., 1988, *Cell Tissue Res.* 254, 317-330). The cells were cultured in MEM medium (214-42, Nacalai Tesque) containing 15% FBS (F-9423, Sigma) and antibiotic-antimycotic (15240-062, GIBCO BRL) for approximately 1 week. Thereafter, the cells were cultured in the aforementioned medium containing 10 nM dexamethasone, 10 mM β-glycerophosphate, and 5 µg/ml vitamin C phosphate for 2 days.

### 3. Experimentation concerning in vivo transplantation into rat

### 3.1 Experimentation concerning subcutaneous transplantation into rat's back

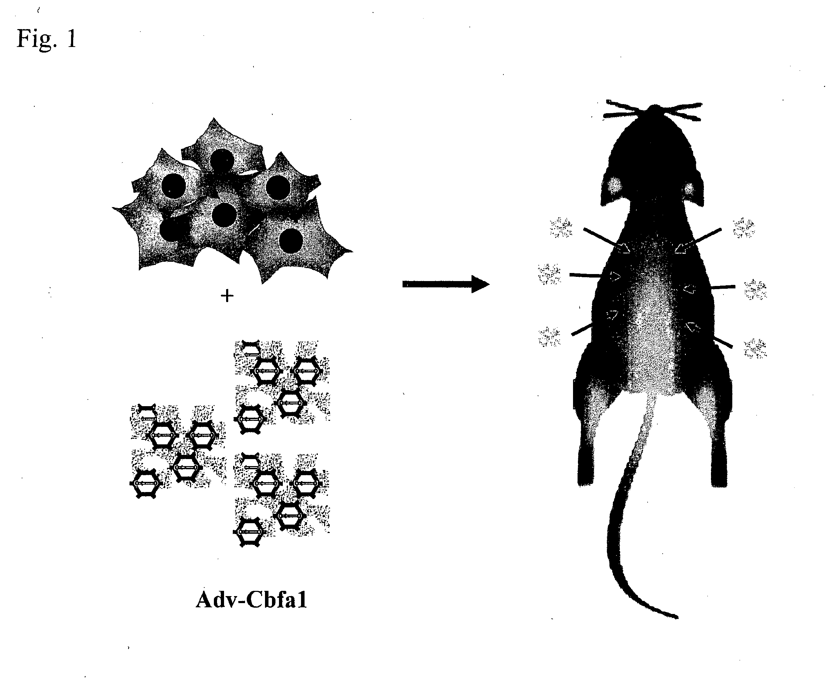

A β-TCP block (2 mm × 2 mm × 2 mm, Olympus) was soaked in a mixed solution of recombinant adenoviruses of Cbfa1 and Cre recombinase genes (1 × 10⁹ pfu/ml) for 3 hours. RBMO harvested by trypsin treatment, the cell density was adjusted to 1,000,000 cells/ml, and the cells were allowed to adsorb onto the block under reduced pressure (100 mmHg). The virus-adsorbed block or an untreated block was subcutaneously transplanted into the rat's back. The block was removed from the rat 3 weeks later and it was then observed by hematoxylin/eosin staining. Fig. 1 schematically shows the experimentation concerning subcutaneous transplantation into rat's back.

### 3.2 Experimentation concerning transplantation into rat femur bone defects

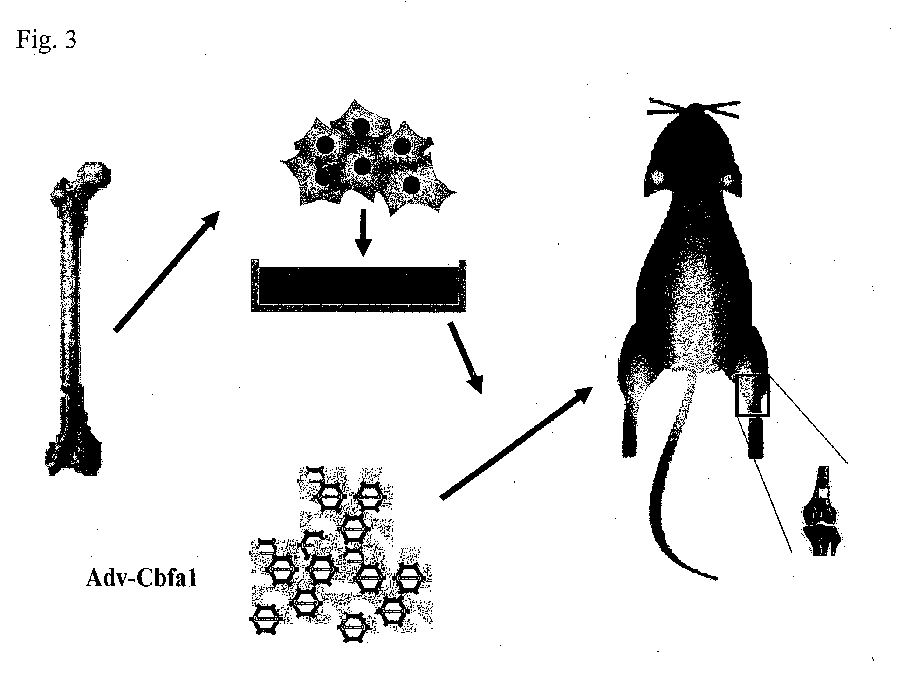

1) A β-TCP block (2 mm × 2 mm × 2 mm, Olympus) or a hydroxyapatite (HA) block (2 mm × 2 mm × 2 mm, Interpore) was soaked in a mixed solution of recombinant adenoviruses of Cbfa1 and Cre recombinase genes (1 × 10⁹ pfu/ml) for 3 hours. The rat femur was perforated using a drill (2.5 mm³) to form a defect, and the block was transplanted thereinto. The virus-adsorbed block or an untreated block was removed from the rat several weeks later and it was then observed by being subjected to hematoxylin/eosin staining.
2) A β-TCP block (2 mm × 2 mm × 2 mm, Olympus) or a hydroxyapatite (HA) block (2 mm × 2 mm × 2 mm, Interpore) was soaked in a mixed solution of recombinant adenoviruses of Cbfa1 and Cre recombinase genes (1 × 10⁹ pfu/ml) for 3 hours. RBMO harvested by trypsin treatment, the cell density was adjusted to 1,000,000 cells/ml, and the cells were allowed to adsorb onto the block under reduced pressure (100 mmHg). The rat femur was perforated using a drill (2.5 mm³) to form a defect, and the virus-adsorbed block or an untreated block was transplanted thereinto. The block was removed from the rat several weeks later and it was then observed by hematoxylin/eosin staining. Fig. 3 schematically shows the experimentation concerning transplantation into rat femur bone defects.

### 4. Preparation and staining of tissue sections

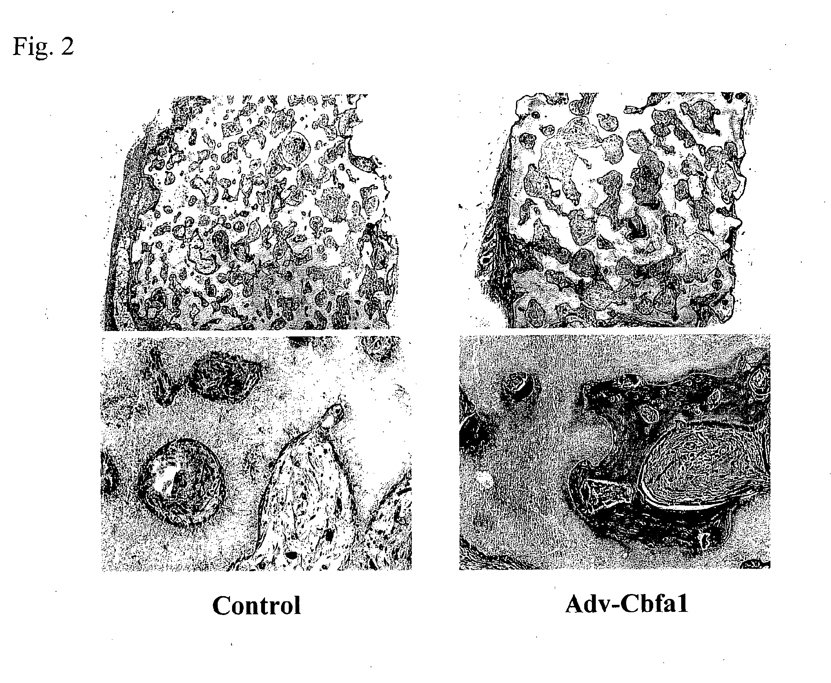

The excised blocks were fixed with 4% paraformaldehyde and 0.05% glutaraldehyde under microwave radiation. On the next day, the resultant was subjected to decalcification in 10% EDTA and 100 mM Tris (pH 7.4), and decalcification was continued for about 1 week. After the decalcification, the blocks were dehydrated in ethanol and lemosol, and then embedded in paraffin. Sections prepared to a thickness of 5 µm each were deparaffinized and stained with hematoxylin and eosin. Results:
1. Results of experimentation concerning subcutaneous transplantation into rat's back
   Fig. 2 shows the results of hematoxylin/eosin staining 3 weeks after subcutaneous transplantation into a rat's back. Regeneration of bone was observed in the center portion of the virus-adsorbed block (Fig. 2: upper right, lower right). In contrast, osteoblast-like cells were observed in the untreated block; however, ossification did not make any progress (Fig. 2: lower left).
2. Results of experimentation concerning transplantation into rat femur bone defects
   1) β-TCP block (2 weeks after transplantation)

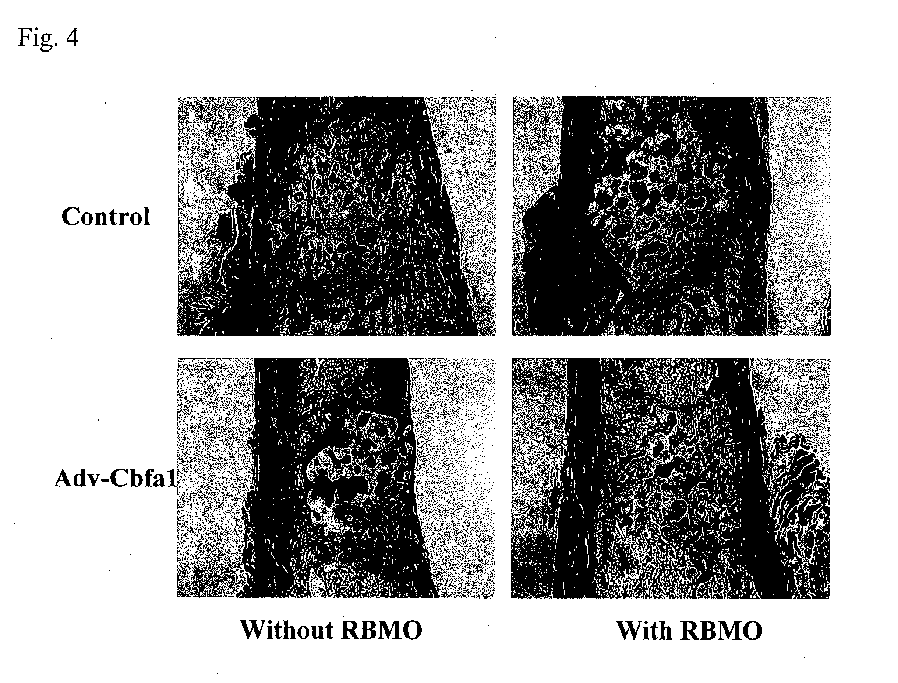

      Fig. 4 shows the results of hematoxylin-eosin staining 2 weeks after transplantation of the β-TCP block into femur bone defects. The degree of resorption of material was more advanced in pores of the Cbfa1 gene recombinant virus-adsorbed block, and ossification was advanced around the pores (Fig. 4: lower left, lower right). Compared with the block to which no osteoblast was added, the degrees of block dissolution and ossification were somewhat advanced in the block to which osteoblasts had been added (Fig. 4: lower right). It was confirmed that adsorption of the Cbfa1 gene recombinant virus enables rapid induction of bone regeneration.
   2) β-TCP block (6 weeks after transplantation)

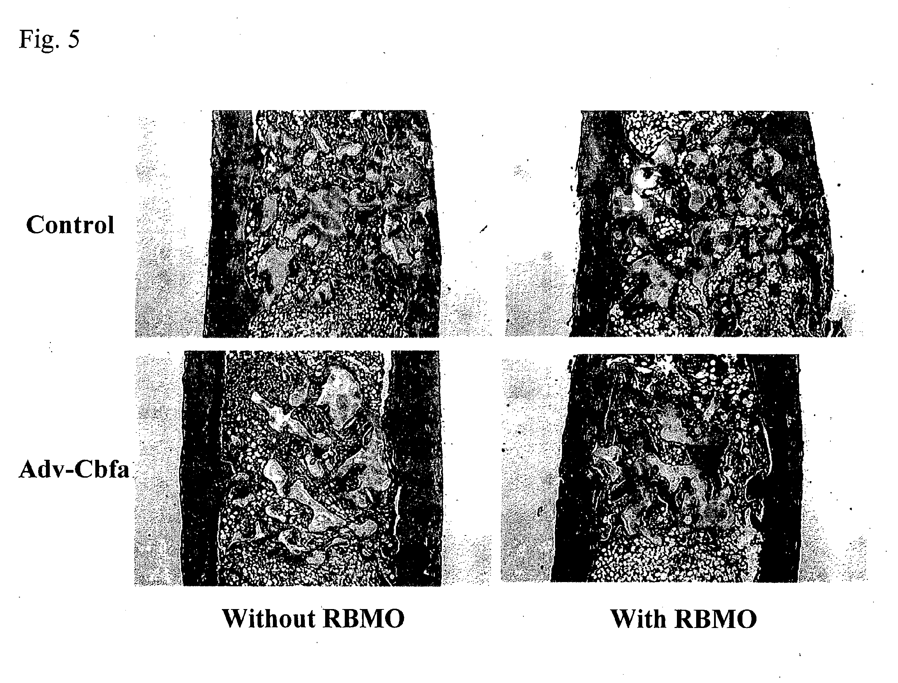

      Fig. 5 shows the results of hematoxylin-eosin staining 6 weeks after transplantation of the β-TCP block into femur bone defects. The degree of resorption of material was more advanced in the Cbfa1 gene recombinant virus-adsorbed block, and the progress of ossification was significant. In particular, rapid bone regeneration in the cortical bone site and rapid block loss in the medullary cavity were observed (Fig. 5: lower left, lower right). The effects of Cbfa1 gene transfection were found to be somewhat stronger in the block to which osteoblasts had been added based on the progress of bone/bone marrow regeneration (Fig. 5: lower right).
   3) HA block (3 weeks after transplantation)

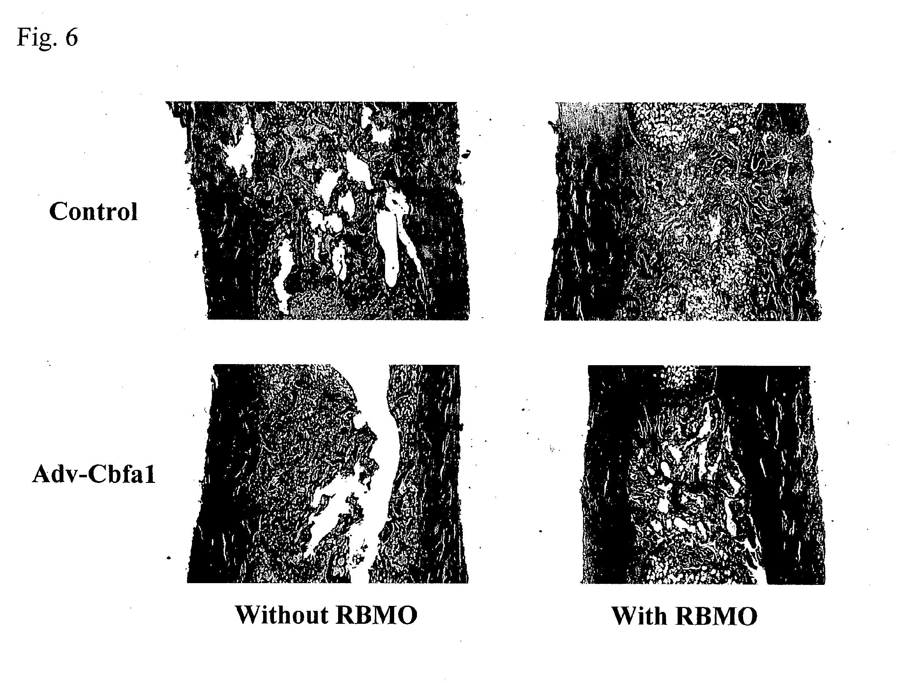

      Fig. 6 shows the results of hematoxylin-eosin staining 6 weeks after transplantation of the HA block into femur bone defects. Compared with the β-TCP block, the HA block used in this experiment has a very large pore size and porosity, which reduces the size of the surface area onto which viruses adsorb. Accordingly, influence of virus adsorption is not significant. Since HA is not a biodegradable material, it is considered that the HA block would remain in the body even after defective portions have been restored.
   4) Comparison of β-TCP block with HA block (3 weeks after transplantation)

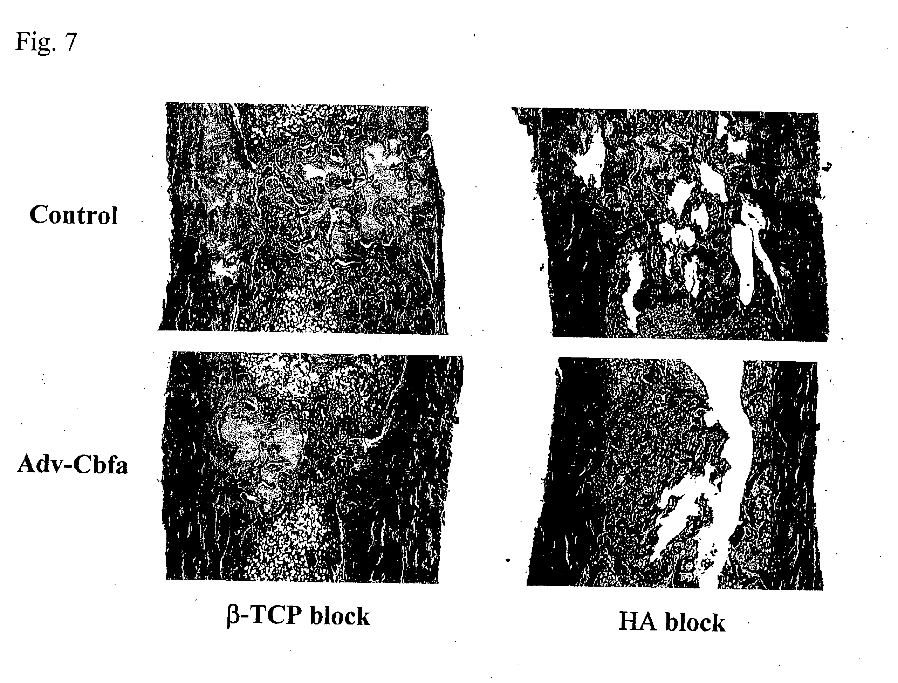

      Fig. 7 shows the results of a comparison of bone regeneration between the HA block and the β-TCP block, which were removed from the body 3 weeks after transplantation. It was found that bone/bone marrow regeneration was promoted with the use of the porous β-TCP block, which is a biodegradable ceramic material, to a greater extent than in the case of the HA block. The effects were observed particularly significantly at the cortical bone site. In the case of the β-TCP block, viruses are gradually diffused throughout the tissues around the graft after transplantation. Accordingly, it was considered that such block influences bone marrow in its vicinity and promotes bone regeneration.
   5) Comparison of virus-adsorbed block with virus-infected cell-adsorbed block (8 weeks after transplantation, β-TCP block)

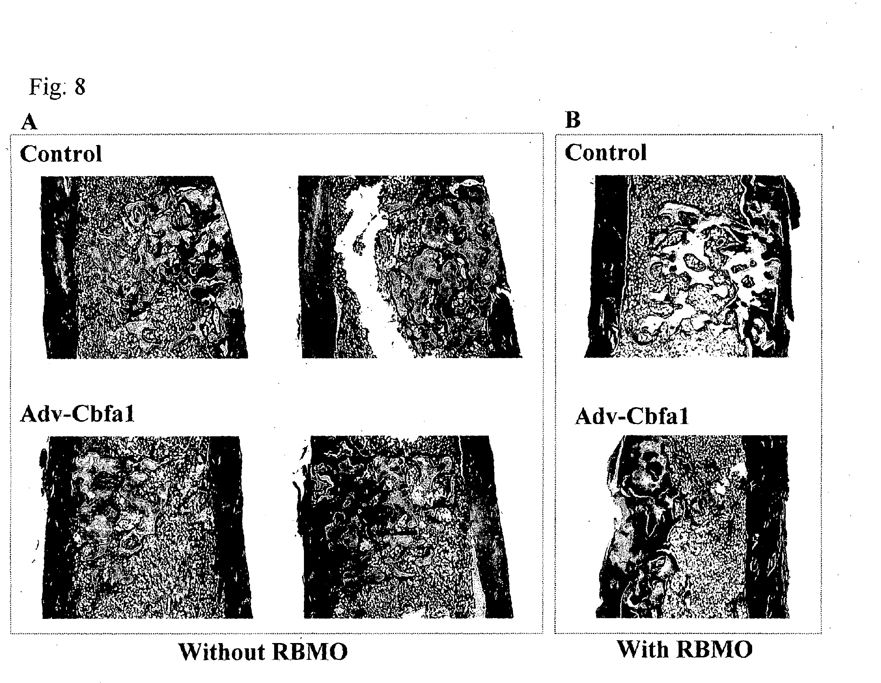

      Fig. 8 shows the results of a comparison of bone regeneration of the virus-adsorbed block and of the virus-infected cell-adsorbed block, which were removed from the body 8 weeks after transplantation. Compared with the virus-infected cell-adsorbed block, dissolution of the block in the medullary cavity and replacement with bone marrow were somewhat insufficient with the use of the virus-adsorbed block. However, progress in ossification at the cortical bone site was equivalent or superior to that observed with the use of the virus-infected cell-adsorbed block.

### Discussion:

Viral vectors carrying Cbfa1 cDNA were allowed to adsorb onto bioadaptable materials, i.e., porous β-TCP and hydroxyapatite (HA), and the resultant was transplanted into the body to allow sustained release of the Cbfa1 gene recombinant viruses. Thus, bone/bone marrow regeneration could be promoted. Based on the progress of bone/bone marrow regeneration, it was considered that the β-TCP block carrying the viral vectors for Cbfa1 gene introduction had effects of bone regeneration equivalent to those of the β-TCP block into which osteoblasts having the Cbfa1 gene adsorbed thereon have been introduced.

Compared with the β-TCP block, the HA block has a very large pore size and porosity, and thus, the surface area onto which viruses adsorb is small. Accordingly, the influence of virus adsorption thereon was not as significant as that on the β-TCP block. However, bone regeneration is found to be more satisfactorily promoted in the presence of the virus-adsorbed carriers, based on the close observation of the cortical bone site in the image showing the stained HA block. Unlike β-TCP, HA is not a biodegradable material and it remains in the body after the restoration of defective portions. In spite of such drawbacks, it was confirmed that HA could be used as a material for the implant of the present invention.

In general, rodents such as rats are less susceptible to adenovirus infection. Thus, the amounts of adenoviruses required for infecting such rodents are 10 times or higher those of adenoviruses required for infecting humans. When adenoviruses are directly introduced into the body, for example, 10⁸ to 10¹⁰ pfu viruses are injected (Okubo et al., Life Science, 2001, vol. 70 (3), 325-36; Trudel et al., *Cancer Gene Therapy,* 2003, vol. 10 (10), 755-763; Hedman et al., Cirvulation, 2003, vol. 107 (21), 2677-2683). In this example, approximately 10 to 20 blocks (2 mm³) were soaked in 1 ml of solution containing 10⁹ pfu/ml viruses, and thus, the amount of viruses introduced into the body was much smaller than 10⁹ pfu. Unlike direct introduction of viruses via injection or other means, viruses are introduced into the body by absorption onto the block according to the method of the present invention. Thus, viruses are less likely to diffuse via the bloodstream after transplantation into the body. Specifically, it was confirmed by the present experimentation that the method of the present invention enables effective bone/cartilage regeneration with the use of a small amount of viruses.

### Example 2: Research concerning sustained release of virus vector using bioadaptable material as carrier (2)

### OPLA composite

### 1. Preparation of adenoviral vector

cDNA was synthesized from the total RNA isolated from murine osteoblasts using AMV reverse transcriptase, PCR was performed with the use of the resulting cDNA as a template and primers specific to Cbfal cDNA, i.e., the sense primer: 5'-ATGCTTCATTCGCCTCACAAAC-3' (SEQ ID NO: 2) and the antisense primer: 5'-TCTGTTTGGCGGCCATATTGA-3' (SEQ ID NO: 3), to amplify Cbfa1 cDNA (GenBank Accession No. AF010284), and the sequence was determined via sequencing. A large quantity of Cbfa1 cDNA was prepared via cloning into the TA cloning vector (pCR II-TOPO, provided by Invitrogen). Cbfa1 cDNA was cleaved with the SpeI and EcoRV restriction enzymes and then blunt-ended.

Mouse VEGF cDNA (GenBank Accession Number NM_009505) was provided by Mr. Watanabe of the Tokyo Institute of Technology. A large quantity of VEGF cDNA was prepared, cleaved with the EcoRI restriction enzyme, and then blunt-ended. Cbfa1 or VEGF cDNA was inserted into the SwaI site of the pAxCALNLw cosmid vector with the use of the Adenovirus Cre/loxP kit (#6151, Takara Shuzo Co., Ltd.) to prepare a recombinant adenovirus (Adv-Cbfa1) vector in accordance with the instructions of the kit. The titers of the resulting viruses were approximately 10¹¹ PFU/ml and approximately 2.5 x 10⁹ PFU/ml. This indicates that the infection efficiency was very high. Since these viruses lacked the E1 regions, they could not proliferate in the target cells, and their actions were transient. Since they had stuffers upstream of the target genes, they allowed gene expression only upon coinfection with Cre recombinase-expressing viruses. The Cre recombinase-expressing adenoviruses (Adv-cre) included in the kit were used.

### 2. Experimentation concerning in vivo transplantation into rat

An experimentation concerning *in vivo* transplantation was performed using the BD 3D OPLA composite (#354614, 5 mm × 3 mm × 0.039 cm², BD Falcon) as a carrier. The BD 3D OPLA (open-cell polylactic acid) composite is a polymer composite synthesized from D, D-L, and L-polylactic acids, and such composite has a polyhedral structure that is effective in high-density suspension culture of cells.

### 2.1 Experimentation concerning subcutaneous transplantation into rat's back

The OPLA composite was soaked in a mixed solution of Adv-Cbfa1 and Ade-cre recombinant adenoviruses (1 × 10⁹ pfu/ml) for 2 minutes during degasification under reduced pressure, and it was then allowed to stand for 3 hours or longer. The OPLA composite with absorption of the viral solution was subcutaneously transplanted into a rat's back. The virus absorbed OPLA composite or an untreated OPLA composite was removed several weeks later and then analyzed.

### 2.2 Experimentation concerning transplantation into rat femur bone defects

The BD 3D OPLA composite (#354614, 5 mm × 3 mm × 0.039 cm², BD Falcon) was used as a bone filler. The OPLA composite was divided into four equal parts under sterile conditions, soaked in a mixed solution of recombinant adenoviruses of Cbfa1 (Adv-Cbfal) or VEGF (Adv-VEGF) and Cre recombinase genes (Adv-cre) (1 x 10⁹ pfu/ml) for 2 minutes during degasification under reduced pressure, and then allowed to stand for 3 hours or longer. The rat femur was perforated using a drill (diameter: 2.5 mm, depth: 2 mm) to form a defect, and the virus absorbed OPLA composite was transplanted thereinto. The virus absorbed OPLA composite or an untreated OPLA composite was removed from the rat several weeks later and then analyzed.

### 3. Preparation and staining of tissue sections

The excised composites were fixed with 4% paraformaldehyde and 0.05% glutaraldehyde under microwave radiation. On the next day, the resultant was subjected to decalcification in 10% EDTA and 100 mM Tris (pH 7.4), and decalcification was continued for about 1 week. After the decalcification, the composites were dehydrated in ethanol, cleared in lemosol, and then embedded in paraffin. Sections prepared to a thickness of 5 µm each were deparaffinized and stained with hematoxylin and eosin. The samples were observed under an optical microscope (IX-70, Olympus, Tokyo, Japan), and the digital images obtained via a CCD camera (CooISNAP cf, Roper Scientific) were analyzed with the use of the MetaMorph software.

Immunostaining was performed in the following manner. Sections prepared to a thickness of 5 µm each were deparaffinized, treated in a proteinase K solution (DakoCytomation, Inc., Glostrup, Denmark) for 5 minutes, and then treated with a peroxidase blocking reagent (DakoCytomation). The sections were treated with a blocking agent (Roche, Basel, Switzerland) to block the binding of nonspecific proteins, and the treated sections were incubated with the mouse monoclonal anti-osteocalcin primary antibody (Zymed Laboratories Inc., San Francisco, CA) overnight at 4°C. The horseradish peroxidase conjugated secondary antibody (Amersham Bioscience, Piscataway, NJ) was added thereto, incubation was further carried out for 1 hour, and color was developed using the DAKO liquid DAB substrate-chromogen (DakoCytomation). Thereafter, hematoxylin was used for nuclear staining. The samples were observed under an optical microscope (IX-70, Olympus, Tokyo, Japan), and the digital images obtained via a CCD camera (CooISNAP cf, Roper Scientific) were analyzed with the use of the MetaMorph software.

### Results:

### 1. Results of experimentation concerning subcutaneous transplantation into rat's back

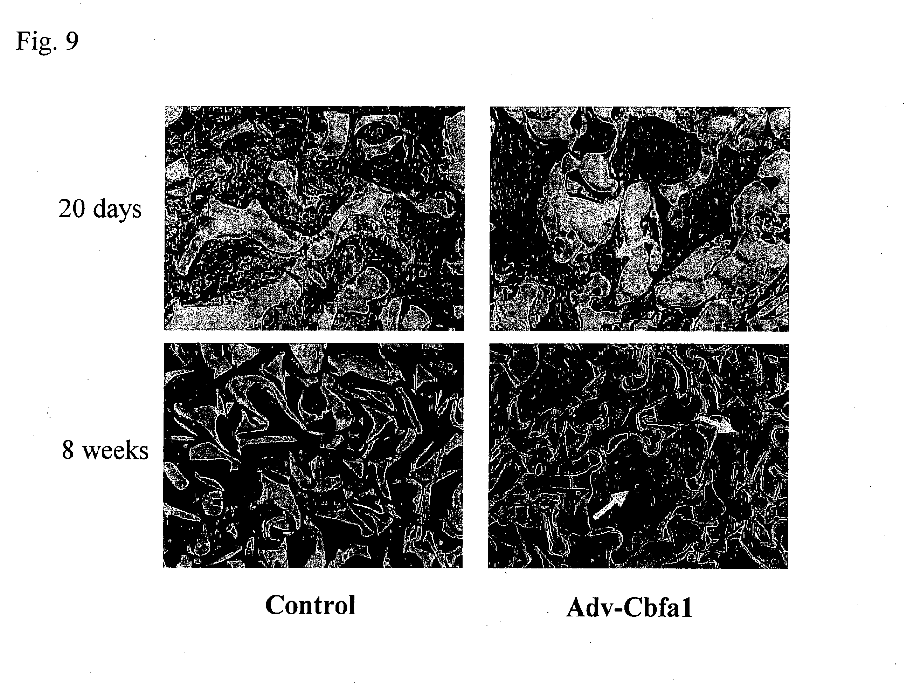

Fig. 9 shows the results of observing images of hematoxylin-eosin staining (20 days and 8 weeks after transplantation). Bone regeneration was observed in the composite carrying Adv-Cbfa1 viruses (the right side of the photograph, indicated by an arrow). In contrast, infiltration of subcutaneous cells such as fat cells was observed, although no ossification was observed in the presence of the control composite (the left side of the photograph, indicated by *).

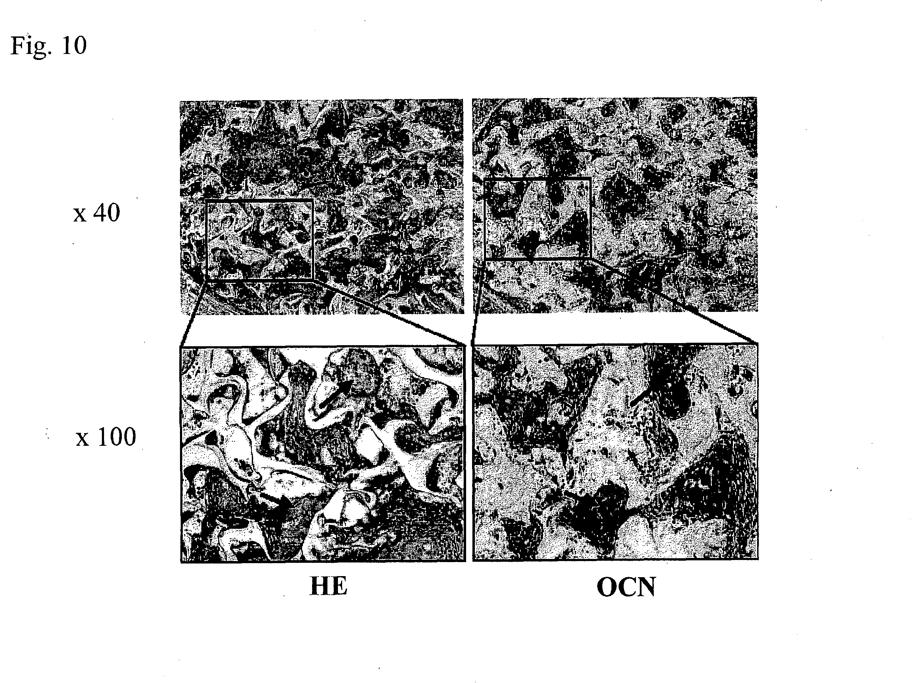

Fig. 10 shows the results of observing the image of osteocalcin immunostaining (20 days after transplantation). The level of osteocalcin expression was high in cells that adhered to the pore surfaces, and expression thereof was observed at a site where calcification occurred. Osteocalcin localization indicates the occurrence of calcification and the regeneration of bone (indicated by an arrow in the photograph).

### 2. Results of experimentation concerning transplantation into rat femur bone defects

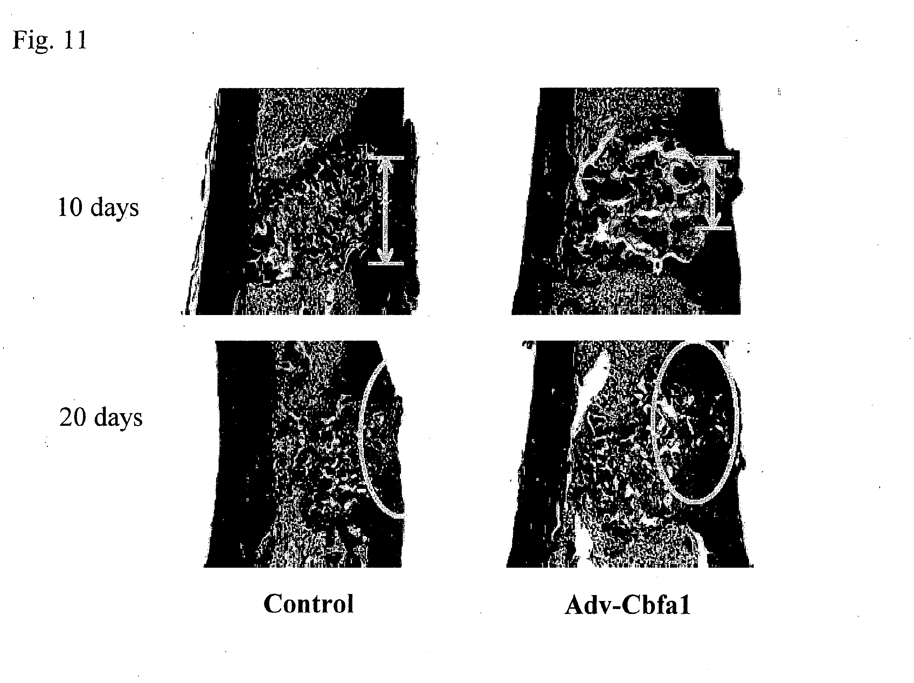

Fig. 11 shows the results of observing images of hematoxylin-eosin staining (10 days and 20 days after transplantation). It was observed that bone regeneration at the cortical bone site was more advanced and the sizes of the cortical bone defects were reduced with the use of the Adv-Cbfa1 absorbed composite (the upper part in the photograph). In the sample 20 days after transplantation, the resorption of the transplanted composite, satisfactory bone regeneration, and fusion between the transplanted composite and the cortical bone were observed (the lower part on the right side in the photograph). In the composite without viruses adsorption (i.e., the control composite), bone regeneration was barely observed 10 days after transplantation, and fusion between the composite and the cortical bone was not significant 20 days after transplantation. These results indicate that the Adv-Cbfa1 virus-adsorbed composite can induce bone regeneration more rapidly, since Adv-Cbfa1 gradually dissolves *in vivo* after transplantation and activates the cells that are present in the vicinity of the composite.

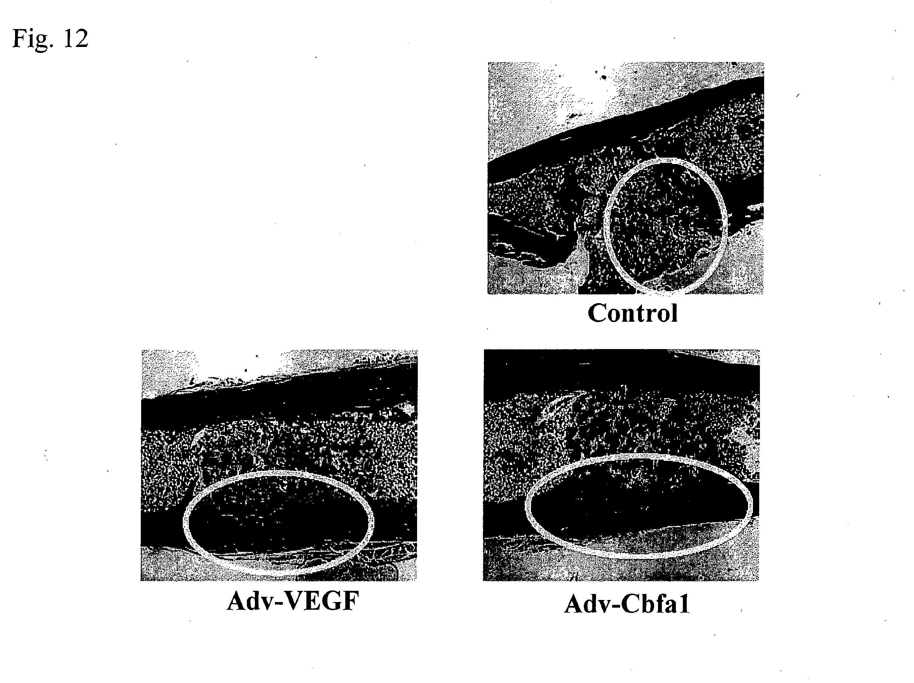

Fig. 12 shows the results of observing the image of hematoxylin-eosin staining (3 weeks after transplantation). The transplanted composites having Adv-Cbfa1 and Adv-VEGF viruses adsorbed thereon were remarkably dissolved and bone regeneration was advanced in the composite (the lower part of the photograph). In particular, rapid bone regeneration at the cortical bone site and fusion between the transplanted composite and the cortical bone were observed (the lower part of the photograph).

As is apparent from the foregoing description, the method of the present invention enables effective bone/cartilage regeneration when the OPLA composite is used as a sustained release carrier.

All publications, patents, and patent applications cited herein are incorporated herein by reference in their entirety.

### Industrial Applicability

The present invention enables bone/cartilage regeneration in safer and simpler manners. The implant according to the present invention can be used as safe and simple means for bone/cartilage regeneration in the fields of dentistry and orthopedics.

### Sequence Listing Free Text

SEQ ID NO: 2- description of artificial sequence: sense primer for Cbfa1 amplification
SEQ ID NO: 3- description of artificial sequence: antisense primer for Cbfa1 amplification

## Claims

1. An implant consisting of a bioadaptable material comprising an adenoviral or retroviral vector carrying a gene of an osteo-/chondro-inducible transcription factor.

2. The implant according to claim 1, wherein the osteo-/chondro-inducible transcription factor is at least one member selected from the group consisting of Cbfa1, Dlx-5, Bapx1, Msx2, Scleraxis, and Sox9.

3. The implant according to claim 1, wherein the osteo-/chondro-inducible transcription factor is Cbfa1.

4. The implant according to any one of claims 1 to 3, wherein the bioadaptable material is any member selected from the group consisting of hydroxyapatite, α-TCP, β-TCP, collagen, polylactic acid, hyaluronic acid, polyglycolic acid, and a complex of any thereof.

5. The implant according to claim 4, wherein the bioadaptable material is β-TCP.
